# FASCICULE DE BREVET EUROPEEN

(11) **EP 1 935 275 B1**
(45) Date de publication et mention de la délivrance du brevet: **03.02.2010**
(21) Numéro de dépôt: 07301718.8
(22) Date de dépôt: 20.12.2007
(51) Int. Cl.: A45D 7/06

(54) **Procédé de déformation permanente des fibres kératiniques comprenant une étape d'application d'une composition réductrice faiblement concentrée et une étape intermediaire de séchage**
Verfahren zur dauerhaften Verformung von Keratinfasern, das eine Anwendungsphase einer gering konzentrierten reduzierenden Zusammensetzung und eine Zwischentrockenphase umfasst
Method for permanently deforming keratinous fibres including a step of applying a reducing composition with a low concentration and an intermediate drying step

(30) Priorité: 22.12.2006 FR 0655915
(43) Date de publication de la demande: 25.06.2008
(73) Titulaire: L'Oréal, 75008 Paris (FR)
(72) Inventeur: Campain, Catherine, 75019, PARIS (FR)
(74) Mandataire: Catherine, Alain

(56) Documents cités:
- EP-A- 1 417 906
- EP-A- 1 535 530
- DE-U1- 8 627 125
- DE-U1- 29 915 590

## Description

La présente invention se rapporte à un procédé de déformation permanente des cheveux comprenant notamment une étape de séchage des cheveux entre une étape d'application d'une composition réductrice, et une étape d'application d'une composition oxydante, ce procédé étant associé à l'utilisation d'un moyen de mise sous tension mécanique de cheveux, ledit moyen étant flexible et refermable sur lui-même, de façon à former une boucle.

La technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, généralement à l'eau, à reconstituer dans un second temps lesdites liaisons disulfures en appliquant, sur les cheveux préalablement mis sous tension par exemple par des bigoudis, une composition oxydante (étape d'oxydation, dite aussi de fixation) de façon à donner finalement aux cheveux la forme recherchée. Cette technique permet ainsi de réaliser l'ondulation des cheveux (procédé de permanente). La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

La traction exercée par les bigoudis permet de réaliser un décollement des cheveux au niveau des racines conduisant à une augmentation du volume de la chevelure, et conduisent, de manière inhérente à une frisure de toute la mèche de cheveux.

Or, de nombreuses femmes souhaitent augmenter le volume de leur chevelure sans pour autant avoir des cheveux frisés. En d'autres termes, s'il est souhaitable de pouvoir réaliser une augmentation du volume de la chevelure, il existe également un besoin pour remplacer la frisure de toute la mèche de cheveux inhérente aux procédés classiques de mise en forme de cheveux qui est essentiellement bidimensionelle, par des boucles rondes en spirales en 3 dimensions.

Il existe donc un besoin pour un procédé de déformation permanente des cheveux permettant de réaliser, de manière durable, une augmentation du volume des cheveux par décollement de ceux-ci au niveau de la racine, associé à des boucles tridimensionelles.

Si la mise en plis avec un fer rond permet d'obtenir ce type de boucles rondes en spirales, celles-ci ne résistent pas au premier shampooing du fait de la rupture des liaisons ioniques et salines créées.

Le document DE 299 15 590 A1 décrit un procédé de traitement des cheveux dans lequel les cheveux lavés et frictionnés sont enroulés autour de rouleaux puis mouillés régulièrement avec un milieu de mise en forme que l'on laisse agir à température ambiante pendant 20 minutes. Les cheveux sont alors rincés à l'eau puis traités avec une solution oxydante. Après enlèvement des rouleaux, les cheveux sont à nouveau rincés à l'eau, mis en plis et ensuite séchés.

L'utilisation de rouleaux classiques au cours du procédé classique de mise en forme des cheveux, n'est pas non plus satisfaisante, puisque ce type de technique conduit a une mauvaise tenue des cheveux dans le temps, et ne permet pas d'aboutir à des boucles tridimensionelles.

Aucun des procédés décrits ne conduit donc à un procédé de déformation permanente totalement satisfaisant vis-à-vis des problèmes mentionnés ci-dessus.

La demanderesse a découvert que les problèmes de l'art antérieur étaient résolu par un procédé de déformation permanente des fibres kératiniques comprenant notamment une étape de séchage des fibres kératiniques entre l'étape d'application d'une composition réductrice et l'étape de fixation par oxydation, ce procédé étant associé à l'utilisation de moyens de mise sous tension mécanique des fibres kératiniques, lesdits moyens étant flexibles et refermables sur eux-mêmes, de façon à former une boucle. L'invention a donc pour objet un procédé de déformation permanente des fibres kératiniques, en particulier des cheveux, comprenant :
- une étape de mise sous tension mécanique des fibres kératiniques par enroulage de celles-ci sur un moyen de mise sous tension mécanique, ledit moyen étant flexible et refermable sur lui-même, de façon à former une boucle, puis
- une étape d'application sur les fibres kératiniques d'une composition réductrice, pour réduire les liaisons disulfure de la kératine, puis après un rinçage éventuel,
- une étape de séchage des fibres kératiniques, puis
- une étape de fixation par oxydation, pour reformer lesdites liaisons, par application sur les fibres kératiniques d'une composition oxydante.

Le procédé défini ci-dessus permet de réaliser, rapidement et de manière durable une augmentation du volume des fibres kératiniques, et en particulier des cheveux par décollement de ceux-ci au niveau de la racine, associé à des boucles tridimensionelles.

Par flexible, on entend au sens de la présente invention, un dispositif comportant en partie ou en totalité de sa longueur un matériau suffisamment souple pour que ledit dispositif puisse se replier sur lui-même et former une boucle. Ce matériau peut être un matériau plastique déformable, moulé, aéré ou expansé, un papier métallisé ou non, un matériau fibreux tel que les cordes et ficelles, un matériau non tissé.

Par refermable, on entend au sens de la présente invention un dispositif permettant de former une boucle sur au moins une partie de sa longueur.

De préférence, le moyen de mise sous tension mécanique est un bigoudi ou une papillote adaptés. De manière tout à fait préférée, le bigoudi utilisé lors de l'étape de mise sous tension des cheveux est un bigoudi tulipe se composant d'une tige allongée formant un corps terminé à une extrémité par une tête dotée d'au moins une perforation. Le corps du bigoudi est réalisé en matière flexible, de sorte que son extrémité libre puisse être introduite dans la perforation de la tête et y être maintenue par serrage élastique. Un bigoudi du type défini ci-dessus, convenant particulièrement à la mise en oeuvre du procédé objet de l'invention est par exemple décrit dans la demande de brevet FR2602650.

L'utilisation d'un bigoudi tulipe permet de contrôler la tension appliquée aux cheveux et permet ainsi de donner une forme arrondie aux mèches de cheveux pendant la durée du traitement, sans traction excessive contrairement à ce que l'on observe avec la plupart des bigoudis cylindriques. L'utilisation d'un ou plusieurs bigoudi(s) tulipe dans le cadre du procédé selon l'invention permet également d'éviter les problèmes de marques à la racine du cheveu et d'obtenir des boucles tridimensionelles en forme de spirale, d'amplitude variable suivant la quantité de bigoudis tulipe utilisés, plutôt qu'une ondulation bidimensionnelle des mèches de cheveux, que l'on observe avec des bigoudis cylindriques. L'enroulage des bigoudis peut se faire sur toute la longueur des cheveux ou à mi-longueur sur des cheveux longs.

Selon la forme de coiffure et la quantité de boucles désirée, l'enroulage se fait avec des mèches plus ou moins épaisses.

Pour obtenir un résultat similaire à celui décrit ci-dessus, une ou des papillottes en papier peuvent également être utilisées à la place des bigoudis tulipe pour peu que les cheveux soient enroulés autour de la papillote et que celle-ci soit refermée sur elle-même pour former une boucle.

La composition réductrice utilisée dans le procédé selon l'invention comprend généralement, dans un milieu cosmétiquement acceptable, au moins un agent réducteur choisi parmi les sulfites, les bisulfites, les thiols et les phosphines.

A titre de sulfites et de bisulfites utilisables, on peut citer les sulfites ou bisulfites des métaux alcalins ou alcalino-terreux ou d'ammonium et en particulier le sulfite ou le bisulfite de sodium, de potassium ou de monoéthanolamine.

De préférence, le ou les thiols utilisés comme agents réducteurs dans la composition réductrice sont choisis parmi la cystéine ses dérivés et ses sels, en particulier la L-cystéine, la D-cystéine, la L,D-cystéine, et leurs sels, la N-acétylcystéine, la cystéamine et ses dérivés, comme ses dérivés acylés en C1-C4 tels que la N-acétyl cystéamine et la N-propionyl cystéamine, l'acide thiolactique ses sels et ses esters, comme le monothiolactate de glycérol, l'acide thioglycolique ses sels et ses esters, comme le monothioglycolate de glycérol ou de glycol, et le thioglycérol, et leurs mélanges.

Comme thiols utilisables dans la composition réductrice utilisée selon l'invention, on peut encore citer les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides tels que ceux décrits dans la demande de brevet EP-A-354 835 et les N-mono- ou N,N-dialkylmercapto 4-butyramides tels que ceux décrits dans la demande de brevet EP-A-368 763, les aminomercaptoalkylamides tels que ceux décrits dans la demande de brevet EP-A-432 000 et les alkylaminomercaptoalkylamides tels que ceux décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Encore plus préférentiellement, la cystéine est utilisée.

Le ou les agents réducteurs représentent de préférence chacun de 0,5 à 10%, de préférence de 1 à 8%, mieux de 2 à 7% en poids par rapport au poids total de la composition réductrice.

Selon un mode de réalisation préféré, on laisse la composition réductrice agir pendant 1 à 50 minutes, de préférence pendant 1 à 30 minutes.

Pendant ou après l'application de la composition réductrice, les fibres kératiniques peuvent être soumises à un traitement thermique par chauffage, par exemple à une température comprise entre 30 et 250°C pendant tout ou partie du temps de pose défini ci-dessus. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants, et en particulier sous film plastique.

Le pH de la composition réductrice selon l'invention est de préférence compris entre 7,5 et 11, de préférence entre 8 et 9,5.

Le pH de la composition réductrice selon l'invention peut être obtenu et/ou ajusté classiquement par ajout soit d'agents alcalins, tels que par exemple l'ammoniaque, la monoéthanolamine, la diéthanolamine, la triéthanolamine, l'isopropanolamine, le 2-méthyl-2-amino-1-propanol, la propanediamine-1,3, un carbonate ou bicarbonate alcalin ou d'ammonium, le carbonate et le bicarbonate de sodium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

Dans le but d'améliorer les propriétés cosmétiques des fibres capillaires ou encore d'atténuer ou d'éviter leur dégradation, la composition réductrice utilisée dans le procédé selon l'invention peut également comprendre un ou plusieurs actifs cosmétiques.

Ce ou ces actifs sont généralement choisis parmi les silicones volatiles ou non, linéaires ou cycliques, aminées ou non, les polymères cationiques, anioniques, non ioniques ou amphotères, les peptides et leurs dérivés, les hydrolysats de protéines, les cires naturelles ou synthétiques, et en particulier les alcools gras, les agents de gonflement et de pénétration ou permettant de renforcer l'efficacité du ou des réducteurs, ainsi que d'autres composés actifs tels que les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents chélatants, les agents opacifiants, les colorants, les filtres solaires, les charges, les vitamines ou provitamines, les huiles minérales, végétales ou synthétiques, ainsi que les parfums, les conservateurs, les stabilisants, et leurs mélanges.

De préférence, la composition réductrice contient au moins un polymère cationique et/ou au moins une silicone

Au sens de la présente demande, "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n ° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 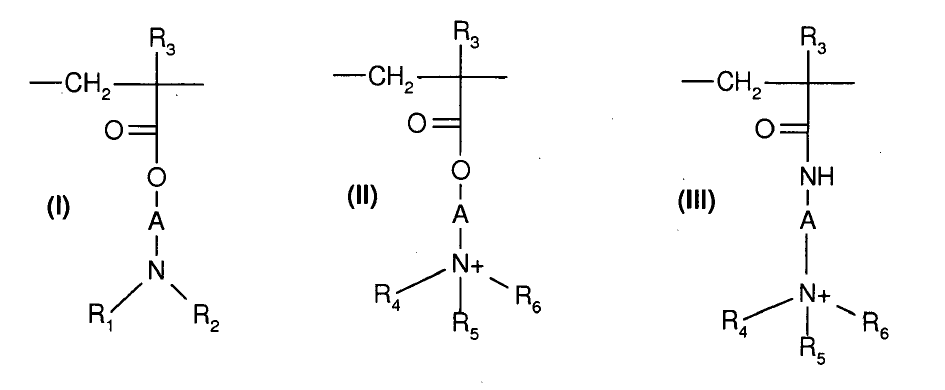 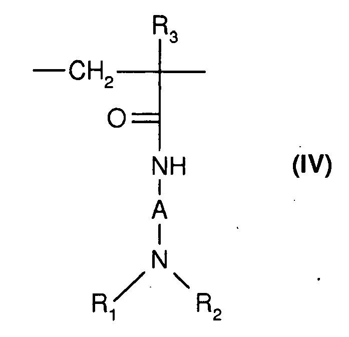 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 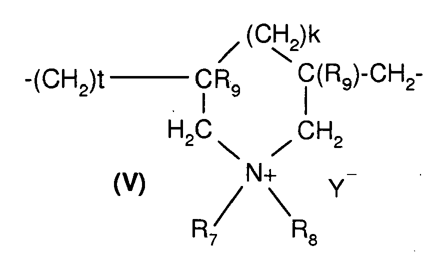 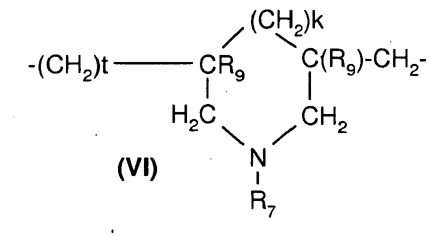 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 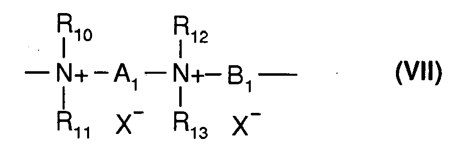 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀,
   R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁,
   R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 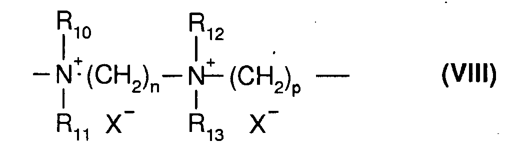 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique. On peut citer en particulier le chlorure d'hexadiméthrine (nom INCI), commercialisé par la société CHIMEX sous la référence MEXOMERE PO ;
(11) Les polyammoniums quaternaires constitués de motifs récurrents de formule (IX) : 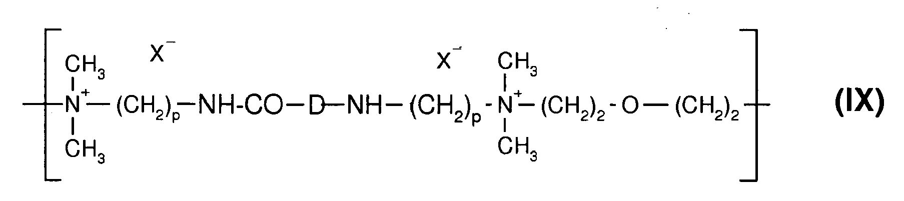 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre un polymère cationique de la famille (10) et par exemple le chlorure d'hexadiméthrine (nom INCI), commercialisé par la société CHIMEX sous la référence MEXOMERE PO.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Comme expliqué précédemment, le ou les actifs cosmétiques pouvant être utilisés dans la composition selon l'invention peuvent être également choisis parmi les silicones.

Les silicones éventuellement présentes dans la composition réductrice selon l'invenion sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylalkylsiloxane, tel que la "SILICONE VOLATILE FZ 3109" commercialisée par la société UNION CARBIDE, de structure chimique (X) : 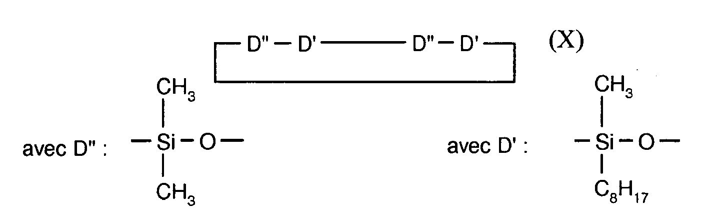 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10-²m²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone pouvant être présentes dans la composition réductrice selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition oxydante du procédé selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition réductrice selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE, les produits 176-12096G de GENERAL ELECTRIC, les produits KF-860, 861 et 864 de SHINETSU ou les produits commercialisés sous les dénominations Q2 8220 ou DCZ-8566 et DOW CORNING 929 ou 939 ou DCZ-8299 ou QZ7224 par la société DOW CORNING. Les groupements aminés substitués sont en particulier des groupements aminoalkyle en C₁-C₄ ; on peut citer les silicones aminées comportant des groupements alcoxy telle que la silicone BELSIL ADM LOG 1 commercialisé par la société WACKER
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

De préférence, la silicone est une silicone aminée.

Comme expliqué précédemment, le procédé selon l'invention comprend une étape d'application d'une composition oxydante.

La composition oxydante comprend généralement au moins un agent oxydant choisi parmi le peroxyde d'hydrogène, le peroxyde d'urée, les bromates alcalins, les polythionates, les persels, tels que les perborates, les percarbonates et les persulfates.

De préférence, l'agent oxydant est le peroxyde d'hydrogène.

Le ou les agents oxydants représentent généralement de 0,1 à 10%, de préférence de 0,5 à 5%, en poids par rapport au poids total de la composition oxydante.

De préférence, lorsque l'agent oxydant est du peroxyde d'hydrogène en solution aqueuse, la composition oxydante utilisée dans le procédé selon l'invention contient au moins un agent stabilisant de l'eau oxygénée.

On peut citer particulier les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.

Le ou les agents stabilisants de l'eau oxygénée représentent généralement de 0,0001% à 5% en poids et de préférence de 0,01 à 2% en poids par rapport au poids total de la composition oxydante.

Généralement, le pH de la composition oxydante varie de 1,5 à 4,5, de préférence de 2 à 3,5.

De préférence, on laisse la composition oxydante agir pendant 2 à 30 minutes, de préférence pendant 2 à 15 minutes, mieux de 2 à 7 minutes.

De préférence, le procédé objet de l'invention comprend une étape d'application d'une composition de soin contenant un polymère cationique.

Tous les polymères cationiques décrits ci-dessus en ce qui concerne la composition réductrice peuvent être utilisés dans la composition de soin.

Parmi tous les polymères cationiques susceptibles d'être utilisés dans le cadre de la présente invention, on préfère mettre en oeuvre dans la composition réductrice les cyclopolymères, en particulier les homolymères du chlorure de diméthyldiallylammonium commercialisé sous la dénomination « MERQUAT® 100 » par la Société MERCK, les polymères de diammonium quaternaire de formule (VIII) ou de formule (IX) et en particulier le MEXOMERE PO.

La silicone préférée est la silicone WACKER BELSIL ADM LOG 1.

Une étape d'application d'une composition de soin permet de limiter ou d'éviter une sensibilisation des cheveux qui pourrait résulter du traitement des cheveux par des agents réducteurs et des agents oxydants au cours du procédé de déformation permanente objet de l'invention. La composition de soin telle que définie ci-dessus permet également de protéger la couleur artificielle des cheveux.

La composition oxydante et la composition de soin utilisées dans le procédé selon l'invention peuvent également comprendre un ou plusieurs actifs cosmétiques, tels que ceux mentionnés précédemment au sujet de la composition réductrice.

Le véhicule des compositions réductrice, oxydante et de soin utilisées dans le procédé selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants organiques peuvent alors être présents dans des concentrations comprises entre environ 0,1 et 20% et, de préférence, entre environ 1 et 10% en poids par rapport au poids total de la composition.

Les pH de la composition réductrice, de la composition oxydante et de la composition de soin utilisées dans le procédé selon l'invention peuvent être obtenus et/ou ajustés classiquement par ajout soit d'agents alcalins, tels que par exemple l'ammoniaque, la monoéthanolamine, la diéthanolamine, la triéthanolamine, l'isopropanolamine, le 2-méthyl-2-amino-1-propanol, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

La composition réductrice, la composition oxydante et la composition de soin utilisées dans le procédé selon l'invention peuvent se présenter indépendamment l'une de l'autre sous forme de lotion, de gel épaissi ou non, de mousse, ou de crème.

Comme expliqué précédemment, le procédé selon l'invention comprend une étape d'application d'une composition réductrice sur les fibres kératiniques, en particulier sur les cheveux, puis une étape d'application d'une composition oxydante.

Selon un mode de réalisation particulier de l'invention, l'étape d'application de la composition réductrice et/ou l'étape de fixation s'effectue(nt) sous chauffage ou est (sont) immédiatement suivie(s) d'un chauffage.

Généralement, le procédé selon l'invention comprend, après l'étape d'application d'une composition réductrice, et/ou après l'étape de fixation par oxydation, une étape de rinçage des fibres kératiniques à l'eau.

La composition réductrice peut également être appliquée au fur et à mesure de l'enroulage des cheveux. Généralement, on laisse ensuite agir la composition réductrice pendant un temps de 1 à 50 minutes, de préférence pendant 1 à 30 minutes.

On peut également, après application de la composition réductrice, soumettre la chevelure à un traitement thermique par chauffage à une température comprise entre 30 et 250°C pendant tout ou partie du temps de pose. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants, et en particulier sous film plastique.

On réalise ensuite éventuellement un rinçage à l'eau des fibres kératiniques, puis on réalise un séchage.

L'étape de séchage est de préférence complète, elle peut être réalisée par exemple avec un sèche-cheveux domestique ou encore un casque ou un bonnet chauffant ou encore être effectuée grâce à un essorage soigneux.

On applique ensuite, sur les cheveux secs, la composition oxydante permettant de reformer les liaisons disulfures de la kératine, généralement pendant un temps de pose de 2 à 30 minutes. On rince ensuite généralement abondamment la chevelure, de préférence à l'eau, puis on enlève les moyens de mise sous tension mécanique présents.

Comme expliqué précédemment, le procédé objet de l'invention peut comprendre une étape d'application d'une composition de soin contenant un polymère cationique et/ou une silicone de préférence une silicone aminée. La composition de soin peut être appliquée à titre d'exemple aux stades suivants du procédé objet de l'invention :
- avant la mise sous tension des cheveux,
- entre l'étape de mise sous tension des cheveux et l'étape d'application de la composition réductrice,
- après l'éventuelle étape de rinçage à l'eau suivant l'étape d'application de la composition réductrice, et avant l'étape de fixation, et/ou
- après l'étape de rinçage à l'eau suivant l'étape de fixation par oxydation.

De préférence, la composition de soin est appliquée avant l'étape de mise sous tension mécanique des fibres kératiniques. Ainsi, de préférence, selon l'invention, l'étape de mise sous tension mécanique des fibres kératiniques est précédée d'une étape d'application d'une composition de soin contenant un polymère cationique et/ou une silicone de préférence une silicone aminée.

De préférence, l'étape d'application d'une composition de soin est suivie d'un rinçage, de préférence d'un rinçage à l'eau.

L'invention a également pour objet un kit associant au moins :
- des moyens de mise sous tension tels que décrits précédemment,
- un premier compartiment contenant une composition cosmétique renfermant au moins un agent réducteur et
- un compartiment contenant une composition cosmétique renfermant au moins un agent oxydant.

L'invention est illustrée par les exemples suivants

### Exemple 1

On prépare une composition réductrice et une composition oxydante pour la mise en oeuvre d'un procédé de déformation permanente des cheveux selon l'invention.

Les formulations sont les suivantes :

### Exemple de composition

Dans les exemples de composition suivants les pourcentages sont exprimés en poids par rapport au poids total de la composition.

### Composition réductrice (1)

| | |
|---|---|
| Cystéine | 4% |
| Thioglycolate d'ammonium en | |
| solution aqueuse à 71% | 1,5% |
| Monoéthanolamine | 2,8% |
| Bicarbonate d'ammonium | 2,7% |
| Cocoamphopropionate de sodium | 1,4% |
| Mexomère PO à 60% de matière active | 1,6% |
| Eau déminéralisée | qsp 100 % |
| | |
| pH 8.8 | |

### Composition oxydante (2)

| | |
|---|---|
| Peroxyde d'hydrogène | 4,8% |
| Stabilisants | 0,03% |
| Acide citrique | 0,1% |
| Merquat 100 (Polyquatermium 6) | 1,25% |
| Oxyde de lauramine | 2,15% |
| Eau déminéralisée | qsp 100% |
| | |
| pH 3 | |

Des essais comparatifs sur tête sur tous types de cheveux, asiatiques, indiens et caucasiens naturels ou sensibilisés ont été réalisés en appliquant les compositions décrites ci-dessus, dans un procédé de déformation conforme à l'invention, décrit dans l'exemple 2. Avec le procédé objet de l'invention, des résultats supérieurs en terme de qualité de boucles ont été obtenus par rapport à un protocole classique. On obtient des boucles tridimensionnelles et un toucher de cheveux très naturel.

De plus, un effet protecteur de la couleur artificielle des cheveux, est observé tant lors de la mise en oeuvre de ce procédé qu'ultérieurement après plusieurs lavages par shampoings. En d'autres termes, le procédé de déformation permanente des fibres kératiniques objet de l'invention ne dégrade pas la coloration artificielle des cheveux.

### Exemple 2 procédé de déformation permanente des cheveux

La chevelure d'un modèle est lavée au moyen d'un shampoing, puis essorée. La chevelure complète est enroulée sur bigoudis tulipes.
La composition réductrice 1 est appliquée, un film plastique est enroulé autour de la tête. Une pause sous casque chauffant est observée pendant 15 minutes.
Le film plastique est enlevé, puis la chevelure est rincée sans enlever les tulipes.
La chevelure est ensuite placée sous casque chauffant jusqu'à séchage complet des cheveux.
La composition oxydante 2 est alors appliquée et une pause de 5 minutes est observée.
Les bigoudis tulipes ou tulipes sont alors enlevées, et la chevelure est rincée.

## Revendications

1. Procédé de déformation permanente des fibres kératiniques, en particulier des cheveux, comprenant :
- une étape de mise sous tension mécanique des fibres kératiniques par enroulage de celles-ci sur un moyen de mise sous tension mécanique, ledit moyen étant flexible et refermable sur lui-même de façon à former une boucle, puis
- une étape d'application sur les fibres kératiniques d'une composition réductrice, pour réduire les liaisons disulfure de la kératine, puis après un éventuel rinçage,
- une étape de séchage des fibres kératiniques, puis
- une étape de fixation par oxydation, pour reformer lesdites liaisons, par application sur les fibres kératiniques d'une composition oxydante.

2. Procédé selon la revendication 1 **caractérisé en ce que** le moyen de mise sous tension mécanique est un bigoudi ou une papillote.

3. Procédé selon la revendication 2 **caractérisé en ce que** le bigoudi est un bigoudi tulipe.

4. Procédé selon l'une quelconque des revendications précédentes, **caractérisé en ce que** la composition réductrice comprend, dans un milieu cosmétiquement acceptable, au moins un agent réducteur choisi parmi les sulfites, les bisulfites, les thiols et les phosphines.

5. Procédé selon la revendication 4, **caractérisé en ce que** l'agent réducteur est choisi parmi la L-cystéine, la D-cystéine, la L,D-cystéine, et leurs sels, l'acide thiolactique ses sels et ses esters, l'acide thioglycolique ses sels et ses esters, et leurs mélanges.

6. Procédé selon la revendication 4 ou 5, **caractérisé en ce que** l'agent réducteur représente de 0,5 à 10%, de préférence de 1 à 8%, mieux de 2 à 7% en poids par rapport au poids total de la composition réductrice.

7. Procédé selon l'une quelconque des revendications précédentes **caractérisé en ce qu'**on laisse la composition réductrice agir pendant 1 à 50 minutes, de préférence pendant 1 à 30 minutes.

8. Procédé selon l'une quelconque des revendications précédentes **caractérisé en ce que** le pH de la composition réductrice est compris entre 7,5 et 11, de préférence entre 8 et 9,5.

9. Procédé selon l'une quelconque des revendications précédentes **caractérisé en ce que** la composition oxydante comprend au moins un agent oxydant choisi parmi le peroxyde d'hydrogène, le peroxyde d'urée, les bromates alcalins, les polythionates, les persels, tels que les perborates, les percarbonates et les persulfates.

10. Procédé selon la revendication 9 **caractérisé en ce que** le ou les agents oxydants représentent généralement de 0,1 à 10%, de préférence de 0,5 à 5%, en poids par rapport au poids total de la composition oxydante.

11. Procédé l'une quelconque des revendications précédentes **caractérisé en ce qu'**on laisse la composition oxydante agir pendant 2 à 30 minutes, de préférence pendant 2 à 15 minutes, mieux de 2 à 7 minutes.

12. Procédé selon l'une quelconque des revendications précédentes **caractérisé en ce que** le pH de la composition oxydante varie de 1,5 à 4,5, de préférence de 2 à 3,5.

13. Procédé selon l'une quelconque des revendications précédentes **caractérisé en ce qu'**il comprend, après l'étape d'application d'une composition réductrice, et/ou après l'étape de fixation par oxydation, une étape de rinçage des fibres kératiniques à l'eau.

14. Procédé selon l'une quelconque des revendications précédentes **caractérisé en ce que** l'étape d'application de la composition réductrice s'effectue sous chauffage ou est immédiatement suivie d'un chauffage.

15. Procédé selon l'une quelconque des revendications précédentes **caractérisé en ce que** la composition réductrice et la composition oxydante se présentent indépendamment l'une de l'autre sous la forme d'une lotion, épaissie ou non, d'une crème, d'un gel ou d'une mousse.

16. Procédé de déformation permanente selon l'une quelconque des revendications précédentes **caractérisé en ce que** l'étape de mise sous tension mécanique des fibres kératiniques est précédée d'une étape d'application d'une composition de soin contenant un polymère cationique et/ou une silicone de préférence une silicone aminée.

17. Procédé de déformation permanente selon la revendication 16 **caractérisé en ce que** l'étape d'application d'une composition de soin est suivie d'un rinçage.

## Claims

1. A method for permanently reshaping the keratin fibers, more particularly for permanently reshaping the hair, comprising:
- a step for mechanically setting the keratin fibers under tension by winding the same around a mechanical tensioning means, said means being flexible and closable upon itself, so as to form a curl, then
- a step for applying onto the keratin fibers a reducing composition, so as to reduce the keratin disulfide bonds, then after an optional rinsing operation,
- a step for drying the keratin fibers, then
- an oxidation fixing step, to reform said bonds, by applying onto the keratin fibers an oxidizing composition.

2. A method according to claim 1, **characterized in that** the mechanical tensioning means is a curler or a curlpaper.

3. A method according to claim 2, **characterized in that** the curler is a tulip-type curler.

4. A method according to any one of the preceding claims, **characterized in that** the reducing composition comprises, in a cosmetically acceptable medium, at least one reducing agent selected from sulfites, bisulfites, thiols and phosphines.

5. A method according to claim 4, **characterized in that** the reducing agent is selected from the group consisting of L-cysteine, D-cysteine, L,D-cysteine, and their salts, thiolactic acid, salts and esters thereof, thioglycolic acid, salts and esters thereof, and mixtures thereof.

6. A method according to claim 4 or 5, **characterized in that** the reducing agent represents from 0.5 to 10%, preferably from 1 to 8%, more preferably from 2 to 7% by weight, as related to the reducing composition total weight.

7. A method according to any one of the preceding claims **characterized in that** the reducing composition is left to stand for 1 to 50 minutes, preferably for 1 to 30 minutes.

8. A method according to any one of the preceding claims, **characterized in that** the pH value of the reducing composition varies from 7.5 to 11, preferably from 8 to 9.5.

9. A method according to any one of the preceding claims, **characterized in that** the oxidizing composition comprises at least one oxidizing agent selected from hydrogen peroxide, carbamide peroxide, alkaline bromates, polythionates, persalts, such as perborates, percarbonates and persulfates.

10. A method according to claim 9, **characterized in that** the oxidizing agent(s) generally represent(s) from 0.1 to 10%, preferably from 0.5 to 5% by weight as related to the oxidizing composition total weight.

11. A method according to any one of the preceding claims, **characterized in that** the oxidizing composition is left to stand for 2 to 30 minutes, preferably for 2 to 15 minutes, more preferably for 2 to 7 minutes.

12. A method according to any one of the preceding claims, **characterized in that** the pH value of the oxidizing composition varies from 1.5 to 4.5, preferably from 2 to 3.5.

13. A method according to any one of the preceding claims, **characterized in that** it comprises, following the reducing composition applying step and/or following the oxidation fixing step, a step consisting in rinsing the keratin fibers with water.

14. A method according to any one of the preceding claims, **characterized in that** the reducing composition applying step is effected under heating or is immediately followed with a heating operation.

15. A method according to any one of the preceding claims, **characterized in that** the reducing composition and the oxidizing composition present independently from each other in the form of a lotion, thickened or not, a cream, a gel or a foam.

16. A method for permanently reshaping according to any one of the preceding claims, **characterized in that** the step for mechanically setting the keratin fibers under tension occurs after a step for applying a hair-care composition which contains a cationic polymer and/or a silicone, preferably, an amino silicone.

17. A method for permanently reshaping method according to claim 16, **characterized in that** the hair-care composition applying step is followed with a rinsing operation.

## Patentansprüche

1. Verfahren zur anhaltenden Verformung von Keratinfasern, insbesondere der Haare, umfassend:
- eine Stufe, bei der Keratinfasern durch Aufwickeln derselben auf ein Mittel zum Anlegen mechanischer Spannung unter mechanische Spannung gebracht werden, wobei das Mittel flexibel ist und derart an sich selbst verschließbar ist, dass eine Schlaufe gebildet wird, danach
- eine Stufe des Auftragens einer reduzierenden Zusammensetzung auf die Keratinfasern, um die Disulfidbindungen des Keratins zu reduzieren, dann nach einem eventuellen Spülen,
- eine Stufe der Trocknung der Keratinfasern, danach
- eine Stufe der Fixierung durch Oxidation, um die Bindungen erneut zu bilden, durch Auftragen einer oxidierenden Zusammensetzung auf die Keratinfasern.

2. Verfahren gemäß Anspruch 1, **dadurch gekennzeichnet, dass** das Mittel zum Anlegen von mechanischer Spannung ein Lockenwickler oder ein Papier-Lockenwickler ist.

3. Verfahren gemäß Anspruch 2, **dadurch gekennzeichnet, dass** der Lockenwickler ein tulpenförmiger Lockenwickler ("bigoudi tulipe") ist.

4. Verfahren gemäß einem der vorangehenden Ansprüche, **dadurch gekennzeichnet, dass** die reduzierende Zusammensetzung in einem kosmetisch verträglichen Medium wenigstens ein Reduktionsmittel umfasst, das aus Sulfiten, Bisulfiten, Thiolen und Phosphinen ausgewählt wird.

5. Verfahren gemäß Anspruch 4, **dadurch gekennzeichnet, dass** das Reduktionsmittel aus L-Cystein, D-Cystein, L,D-Cystein und deren Salzen, Thiomilchsäure, ihren Salzen und ihren Estern, Thioglycolsäure, ihren Salzen und ihren Estern und deren Gemischen ausgewählt wird.

6. Verfahren gemäß Anspruch 4 oder 5, **dadurch gekennzeichnet, dass** das Reduktionsmittel 0,5 bis 10 Gew.-%, vorzugsweise 1 bis 8 Gew.-%, besser 2 bis 7 Gew.-%, bezogen auf das Gesamtgewicht der reduzierenden Zusammensetzung, darstellt.

7. Verfahren gemäß einem der vorangehenden Ansprüche, **dadurch gekennzeichnet, dass** man die reduzierende Zusammensetzung während 1 bis 50 min, vorzugsweise während 1 bis 30 min, einwirken lässt.

8. Verfahren gemäß einem der vorangehenden Ansprüche, **dadurch gekennzeichnet, dass** der pH der reduzierenden Zusammensetzung zwischen 7,5 und 11, vorzugsweise zwischen 8 und 9,5, liegt.

9. Verfahren gemäß einem der vorangehenden Ansprüche, **dadurch gekennzeichnet, dass** die oxidierende Zusammensetzung wenigstens ein Oxidationsmittel umfasst, das ausgewählt wird aus Wasserstoffperoxid, Harnstoffperoxid, Alkalibromaten, Polythionaten, Persalzen wie Perboraten, Percarbonaten und Persulfaten.

10. Verfahren gemäß Anspruch 9, **dadurch gekennzeichnet, dass** das Oxidationsmittel oder die Oxidationsmittel im Allgemeinen 0,1 bis 10 Gew.-%, vorzugsweise 0,5 bis 5 Gew.-%, bezogen auf das Gesamtgewicht der oxidierenden Zusammensetzung, darstellen.

11. Verfahren gemäß einem der vorangehenden Ansprüche, **dadurch gekennzeichnet, dass** man die oxidierende Zusammensetzung während 2 bis 30 min, vorzugsweise während 2 bis 15 min, besser 2 bis 7 min, einwirken lässt.

12. Verfahren gemäß einem der vorangehenden Ansprüche, **dadurch gekennzeichnet, dass** der pH der oxidierenden Zusammensetzung von 1,5 bis 4,5, vorzugsweise von 2 bis 3,5, variiert.

13. Verfahren gemäß einem der vorangehenden Ansprüche, **dadurch gekennzeichnet, dass** es nach der Stufe des Auftragens einer reduzierenden Zusammensetzung und/oder nach der Stufe der Fixierung durch Oxidation eine Stufe des Spülens der Keratinfasern mit Wasser umfasst.

14. Verfahren gemäß einem der vorangehenden Ansprüche, **dadurch gekennzeichnet, dass** die Stufe des Auftragens der reduzierenden Zusammensetzung unter Erwärmen erfolgt oder unmittelbar danach ein Erwärmen erfolgt.

15. Verfahren gemäß einem der vorangehenden Ansprüche, **dadurch gekennzeichnet, dass** die reduzierende Zusammensetzung und die oxidierende Zusammensetzung sich unabhängig voneinander in Form einer verdickten oder nicht verdickten Lotion, einer Creme, eines Gels oder eines Schaums präsentieren.

16. Verfahren zur anhaltenden Verformung gemäß einem der vorangehenden Ansprüche, **dadurch gekennzeichnet , dass** der Stufe, bei der Keratinfasern unter Spannung gebracht werden, eine Stufe des Auftragens einer Pflegezusammensetzung vorausgeht, die ein kationisches Polymer und/oder ein Silicon, vorzugsweise ein aminiertes Silicon, enthält.

17. Verfahren zur anhaltenden Verformung gemäß Anspruch 16, **dadurch gekennzeichnet, dass** auf die Stufe des Auftragens einer Pflegezusammensetzung ein Spülen folgt.
